# EUROPEAN PATENT SPECIFICATION

(11) **EP 3 512 356 B1**
(45) Date of publication and mention of the grant of the patent: **24.04.2024**
(21) Application number: 17777493.2
(22) Date of filing: 13.09.2017
(51) Int. Cl.: A23L 33/00, A23L 33/18, A23L 33/185, A61P 37/08

(54) **INFANT FORMULA FOR COW'S MILK PROTEIN ALLERGIC INFANTS**
BABYMILCH FÜR KUHMILCHPROTEIN ALLERGISCHE SÄUGLINGE
FORMULE INFANTILE POUR NEONATES ALLERGIQUES AU PROTEINES DU LAIT DE VACHE

(30) Priority: 13.09.2016 US 201662393781 P
(43) Date of publication of application: 24.07.2019
(73) Proprietor: Société des Produits Nestlé S.A., 1800 Vevey (CH)
(72) Inventor: SCHUH, Susanne, 3073 Gümlingen (CH); THEVENIER, Anne, 3012 Bern (CH); RAN-RESSLER, Rinat, Bridgewater NJ 08807 (US); RADE-KUKIC, Koraljka, Lafayette, California 94549 (US); KUSLYS, Martinas, 3506 Grosshoechstetten (CH)
(74) Representative: Elkington and Fife LLP
(86) International application number: PCT/EP2017/073041
(87) International publication number: WO 2018/050705

(56) References cited:
- US-A1- 2010 040 591
- US-A1- 2013 115 336
- US-A1- 2015 305 359
- US-A1- 2016 037 817
- US-B1- 6 365 218
- LOKRA ET AL: "Chemical characterization and functional properties of a potato protein concentrate prepared by large-scale expanded bed adsorption chromatography", LWT- FOOD SCIENCE AND TECHNOLOGY, ACADEMIC PRESS, UNITED KINGDOM, vol. 41, no. 6, 12 March 2008 (2008-03-12) , pages 1089-1099, XP022525004, ISSN: 0023-6438, DOI: 10.1016/J.LWT.2007.07.006

## Description

### FIELD OF THE INVENTION

The present invention relates to nutritional compositions for infants. In particular, the invention relates to infant formulas that are suitable for infants with cow's milk protein allergy.

### BACKGROUND TO THE INVENTION

Human breast milk and breast feeding are considered to be the optimal form of nutrition for healthy infants during the first months of life. However, there is a need for nutritional sources that can be used in addition to breast milk. Furthermore, not all infants can be breast fed and the needs of more vulnerable infants, such as preterm infants, cannot be achieved by their mother's milk, so there is also a need for alternatives to breast milk.

Nutritional compositions that satisfy the nutritional requirements of infants may be used as a substitute for or complement to human breast milk. Preferably, infant formulas should have an acceptable taste, and be hypoallergenic when targeted to infants who are allergic or at risk of allergy.

Infant formulas are typically formulated with cow's milk protein. For example, bovine whey protein and/or casein are often used as the protein source in infant formulas. However, some infants exhibit an allergy to cow's milk proteins, making such formulas unsuitable. Allergies to cows' milk and to infant formulas containing cow's milk protein may be due to the differences between the proteins in cows' milk and those in human milk. The principal recognised cow's milk allergens are alpha-lactalbumin (aLA), beta-lactoglobulin (bLG) and bovine serum albumin (BSA).

To reduce allergenicity, cow's milk proteins may be hydrolysed (e.g. enzymatically) either partially, or in the case of products intended for the management of Cow's Milk Protein Allergy (CMPA), extensively. However, such proteins must be highly processed to provide sufficient hydrolysis to reduce the risk of an allergic reaction. Such processing may be viewed unfavourably with an increasing tendency to provide more natural diets and a strong hydrolysis process also tends to have a negative impact on taste. In addition, the extensive processing increases the cost of the product formulas.

Alternatives to cow's milk protein may be used in nutritional compositions, for example soy and rice proteins. However, soy-based nutritional compositions are not recommended by the European Society for Paediatric Gastroenterology, Hepatology and Nutrition (ESPGHAN) for infants (0-12 months), because of the risk of a cross allergic response. Rice-based nutritional compositions require the addition of numerous free amino acids to provide the correct amino acid profile for infant formulas, due to the incomplete natural amino acid distribution in rice proteins. This increases cost and may provide the resulting formula with a less palatable taste. Furthermore, rice proteins are generally insoluble and require at least partial hydrolysis for solubilisation. Compositions for the management of symptoms of cow's milk allergy in paediatric subjects that comprise non-dairy proteins are disclosed in e.g., US 2015/305359 A1. The preparation of native potato protein isolates and their uses in food, nutraceutical and pharmaceutical products is disclosed in US 2010/040591 A1.

Infant formulas may be formulated entirely from free amino acids for infants with severe cases of multiple allergies. However, ESPGHAN guidelines indicate that such formulas should not be used as a first line solution in the case of cow's milk protein allergic infants. Furthermore, overprescription of amino acid based formulas adds to the cost burden on national health systems as amino acid based formulas are even more expensive than extensively hydrolysed formulas. Accordingly, there is a significant need for nutritional compositions for infants that comprise less potential allergens, and preferably which require minimal processing, have good taste and have low cost. In particular, there is a need for infant formulas that are suitable for administration to infants with cow's milk protein allergy.

### SUMMARY OF THE INVENTION

The inventors have developed a nutritional composition based on potato protein as the major protein source, which is naturally absent in the major allergens found in milk and soy. Accordingly, the nutritional composition may provide a naturally hypoallergenic infant formula that is suitable for infants with cow's milk protein allergy.

The inventors have found potato protein to have a well balanced amino acid profile, which is closer to that of human milk than rice or soy protein. Accordingly, less addition of free amino acids is required to provide a composition with the required nutritional profile, which renders the resulting product more cost effective and gives it a more palatable taste.

Moreover, as a result of their lower allergen profile, the potato protein components do not require extensive hydrolysis, which provides significant benefits in terms of cost and for the development of the infant, because the intact or slightly hydrolysed proteins facilitate improved gut maturation.

Furthermore, the need for an emulsifier may be reduced or removed, because the potato protein itself may provide any necessary emulsifier properties. In addition, use of potato protein provides for good acceptance, for example in terms of taste and texture of the nutritional composition.

Accordingly, in one aspect the invention provides an infant formula comprising protein, carbohydrate and fat, wherein the major source of protein is potato protein, wherein the potato protein has a molecular mass less than 35 kDa and wherein the carbohydrate comprises lactose, saccharose, maltodextrin or starch.

In another aspect the invention provides an infant formula comprising protein, carbohydrate and fat, wherein the major source of protein is potato protein, wherein the potato protein has a molecular mass greater than 35 kDa and wherein the carbohydrate comprises lactose and optionally saccharose, maltodextrin or starch.

In one embodiment, the infant formula is for an infant with cow's milk protein allergy.

In a particularly preferred embodiment, the infant formula does not comprise dairy protein.

In a preferred embodiment, the major source of protein in the nutritional composition is potato protein and the remaining protein is plant protein.

The term "major source of protein is potato protein" means that the largest fraction of the total protein by weight in a composition originates from potato protein.

In one embodiment, at least about 50%, 55%, 60%, 65%, 70%, 75%, 80%, 85%, 90% or 95%, preferably 75%, by weight of the total protein is potato protein.

In a preferred embodiment, 100% by weight of the total protein is potato protein.

In a preferred embodiment, the protein (in particular, the potato protein) is intact protein. Preferably, the protein has not been subjected to artificial hydrolysis.

In another embodiment, the protein (in particular, the potato protein) is partially hydrolysed protein.

In another embodiment, the protein (in particular, the potato protein) is extensively hydrolysed protein.

In one embodiment, the infant formula further comprises free amino acids.

In one embodiment, the infant formula does not comprise a further emulsifier. The potato protein may provide sufficient function as an emulsifier.

In one embodiment, the infant formula is in the form of a powder or liquid. The liquid may be, for example, a concentrated liquid infant formula or a ready-to-feed formula. In one embodiment, the infant formula is in the form of a reconstituted infant formula (i.e. a liquid infant formula that has been reconstituted from the powdered form). Preferably, the infant formula is in the form of a powder.

In one embodiment, the infant formula further comprises lactose. In one embodiment, the infant formula does not comprise lactose.

In one embodiment, the infant formula further comprises probiotics. In one embodiment, the infant formula does not comprise probiotics.

In one embodiment, the infant formula further comprises nucleotides. In one embodiment, the infant formula does not comprise nucleotides.

In one embodiment, the infant formula comprises:
(a) 1.8-3.2 g protein per 100 kcal;
(b) 9-14 g carbohydrate per 100 kcal; and
(c) 4.0-6.0 g lipids per 100 kcal.

In another aspect, the invention provides the use of potato protein for the manufacture of an infant formula, wherein the major source of protein in the infant formula is potato protein.

The infant formula may be as disclosed herein.

In another aspect, also disclosed but not part of the present invention, is a method of feeding an infant comprising administering to the infant the infant formula of the invention.

In a preferred embodiment, the infant has cow's milk protein allergy.

In another aspect, the invention provides the infant formula of the invention for use in a method of treatment of cow's milk protein allergy in an infant wherein said infant is fed said infant formula.

In another aspect, the invention provides a method of making an infant formula comprising the step of admixing potato protein with at least one other component of the infant formula.

The infant formula may be as disclosed herein.

### DESCRIPTION OF THE DRAWINGS

**Figure 1**
   Comparison of essential amino acid levels between potato and rice protein, and FAO 2013 recommendations.
**Figure 2**
   Comparison of essential amino acid levels between potato and rice protein, and FAO 2013 recommendations.
**Figure 3**
   Comparison of histidine levels between potato and rice protein, and Institute of Medicine of the National Academies recommendations (assuming an infant formula intake of 1000 mL per day, with a minimum of 1.8 g protein per 100 kcal for infants of 6 months of age (or 12.6 g protein per day)).
**Figure 4**
   Comparison of isoleucine, leucine, lysine and tryptophan levels between potato and rice protein, and Institute of Medicine of the National Academies recommendations (assuming an infant formula intake of 1000 mL per day, with a minimum of 1.8 g protein per 100 kcal for infants of 6 months of age (or 12.6 g protein per day)).
**Figure 5**
   Comparison of branched-chain amino acid (BCAA) levels between potato and rice protein, and whole milk.
**Figure 6**
   Comparison of mean levels of threonine and lysine between potato and rice protein, and FAO 2013 recommendations.
**Figure 7**
   Comparison of mean levels of combined aromatic amino acids between potato and rice protein, and whole milk.

### DETAILED DESCRIPTION OF THE INVENTION

Various preferred features and embodiments of the present invention, which is defined by the claims, will now be described by way of non-limiting examples.

The practice of the present invention will employ, unless otherwise indicated, conventional techniques of chemistry, biochemistry, molecular biology, microbiology and immunology, which are within the capabilities of a person of ordinary skill in the art. Such techniques are explained in the literature. See, for example, Sambrook, J., Fritsch, E.F. and Maniatis, T. (1989) Molecular Cloning: A Laboratory Manual, 2nd Edition, Cold Spring Harbor Laboratory Press; Ausubel, F.M. et al. (1995 and periodic supplements) Current Protocols in Molecular Biology, Ch. 9, 13 and 16, John Wiley & Sons; Roe, B., Crabtree, J. and Kahn, A. (1996) DNA Isolation and Sequencing: Essential Techniques, John Wiley & Sons; Polak, J.M. and McGee, J.O'D. (1990) In Situ Hybridization: Principles and Practice, Oxford University Press; Gait, M.J. (1984) Oligonucleotide Synthesis: A Practical Approach, IRL Press; and Lilley, D.M. and Dahlberg, J.E. (1992) Methods in Enzymology: DNA Structures Part A: Synthesis and Physical Analysis of DNA, Academic Press. Each of these general texts is herein incorporated by reference.

### Infant

The subjects referred to in the present disclosure as the target of the nutritional compositions disclosed herein are human subjects.

The term "infant" refers to a child under the age of 12 months, for example a child between 0 and 6 months of age.

### Allergy

The term "allergy" refers to a hypersensitivity of the immune system to a substance which is normally tolerated. The allergy may be an allergy detected by a medical doctor.

The term "food allergy" refers to an allergy with respect to a nutritional composition.

Infant formulas are typically formulated with cow's milk protein. For example, bovine whey protein and/or casein are often used as the protein source in infant formulas. However, some infants exhibit an allergy to cow's milk proteins, making such formulas unsuitable.

Allergies to cows' milk and to infant formulas containing cow's milk protein may be due to the differences between the proteins in cows' milk and those in human milk. The principal recognised cow's milk allergens are alpha-lactalbumin (aLA), beta-lactoglobulin (bLG) and bovine serum albumin (BSA).

### infant formula

The term "infant formula" may refer to a foodstuff intended for particular nutritional use by infants during the first year of life and satisfying by itself the nutritional requirements of this category of person, as defined in European Commission Directive 2006/141/EC of 22 December 2006.

Infants can be fed solely with infant formulas or the infant formula can be used as a complement of human milk.

The term "infant formula" includes hypoallergenic infant formulas. A hypoallergenic composition is a composition which is unlikely to cause allergic reactions.

The infant formula of the invention may be in the form of a powder or liquid. The liquid may be, for example, a concentrated liquid infant formula or a ready-to-feed formula. The infant formula may be in the form of a reconstituted infant formula (i.e. a liquid infant formula that has been reconstituted from the powdered form). Preferably, the infant formula is in the form of a powder.

The powder is preferably capable of being reconstituted into a liquid composition suitable for feeding an infant, for example by the addition of water. Similarly, the concentrated liquid infant formula is preferably capable of being diluted into a liquid composition suitable for feeding an infant, for example by the addition of water.

In one embodiment, the infant formula has an energy density of about 60-70 kcal per 100 mL, when formulated as instructed.

### Protein

The term "protein" refers to polymers of amino acids, and includes polypeptides and peptides. The term "protein" does not encompass free amino acids, which may also be present in the infant formula of the invention.

The protein content of the infant formula of the invention is preferably in the range 1.8-3.2 g protein per 100 kcal. In a preferred embodiment, the protein content of the infant formula of the invention is in the range 1.8-2.8 g protein per 100 kcal.

The infant formula of the invention comprises potato protein as the major protein source.In one embodiment, at least about 50%, 55%, 60%, 65%, 70%, 75%, 80%, 85%, 90% or 95%, preferably at least about 75%, more preferably 100%, by weight of the total protein is potato protein.

The remaining protein in the infant formula of the invention may be any protein which is suitable for use in infant formula.

Preferably, the infant formula does not comprise dairy protein. Accordingly, in a preferred embodiment 100% by weight of the total protein is non-dairy protein.

In a preferred embodiment, 100% by weight of the total protein is plant protein.

Example plant proteins that may optionally be used in the infant formula of the invention, in addition to the potato protein, include, pea, rice, quinoa, oat, sunflower or coconut proteins, or combinations thereof.

Further example non-dairy proteins for use in the infant formula of the invention include algal protein or leaf protein.

In a preferred embodiment, the major source of protein in the infant formula is potato protein and the remaining protein is plant protein.

In a preferred embodiment, 100% by weight of the total protein is potato protein.

Potato protein for use in the infant formulas of the invention is readily accessible or available, for example as concentrates or isolates, for example from commercial sources.

Potato protein may be extracted from potato tuber juice, which may itself be separated from potato solids by any of a number of suitable techniques known in the art. Chromatographic techniques may be used to purify potato proteins from the tuber juice in a similar manner to the isolation of milk proteins. Once isolated, the potato protein may be concentrated and subjected to temperature treatment and/or pH adjustment. Further steps may include, for example, removal of triglycoalkaloids, spray drying and/or UV treatment.

Suitable potato protein sources include potato protein fractionated by molecular mass, in one embodiment having a high molecular molecular mass of greater than 35 kD, and in another embodiment, the potato protein source is a low molecular mass potato protein fraction of less than 35 kDa.

The protein may be, for example, intact protein or hydrolysed protein (e.g. partially hydrolysed protein). Preferably, the protein is intact protein.

Hydrolysis of protein may in general be termed "partial" or "extensive" depending on the degree to which hydrolysis is carried out. Protein hydrolysates may have an extent of hydrolysis that is characterised by NPN/TN%, which refers to the non-protein nitrogen divided by the total nitrogen × 100. The non-protein nitrogen refers to amino nitrogen that is free to react with a reagent such as trinitrobenzenesulfonic acid (TNBS). NPN/TN% may be measured as described in Adler-Nissen (Adler-Nissen, J. (1979) J. Agric. Food Chem. 27: 1256-1262).

The term "extensive hydrolysis" may refer to hydrolysis that provides protein that has a NPN/TN% greater than 95%. The term "partial hydrolysis" may refer to hydrolysis that provides protein that has a NPN/TN% in the range 70-85%.

In one embodiment, the protein has an NPN/TN% between 5-90%, 70-90% or 70-85%, preferably between 70-85%.

In one embodiment, 60-70% of the protein population has a molecular mass of less than 5000 Da.

In another embodiment, the protein has an NPN/TN% greater than 95%. These are "extensive" hydrolysates.

In one embodiment, 60-70% of the protein population has a molecular mass of less than 3000 Da. In one embodiment, at least 95% of the protein population has a molecular mass of less than 3000 Da.

Proteins for use in the infant formula of the invention may be hydrolysed by any suitable method known in the art. For example, proteins may enzymatically hydrolysed, for example using a protease.

For example, protein may be hydrolysed using alcalase (e.g. at an enzyme:substrate ratio of about 2-15% by weight and for a duration of about 1-5 hours).

### Free amino acids

The infant formulas of the invention may further comprise free amino acids. Such free amino acids provide a protein equivalent source.

Free amino acids may be incorporated in the infant formulas of the invention to supplement the amino acids comprised in the protein. The levels of free amino acids may be chosen to provide an amino acid profile that is sufficient for infant nutrition, in particular an amino acid profile that satisfies nutritional regulations (e.g. European Commission Directive 2006/141/EC).

Example free amino acids for use in the infant formula of the invention include histidine, isoleucine, leucine, lysine, methionine, cysteine, phenylalanine, tyrosine, threonine, tryptophan, valine, alanine, arginine, asparagine, aspartic acid, glutamic acid, glutamine, glycine, proline, serine, carnitine, taurine and mixtures thereof.

### Carbohydrate

The carbohydrate content of the infant formula of the invention is preferably in the range 9-14 g carbohydrate per 100 kcal.

The carbohydrate may be any carbohydrate which is suitable for use in infant formula.

The infant formula of the present invention comprise carbohydrate, said carbohydrate comprising lactose, saccharose, maltodextrin or starch. Mixtures of carbohydrates may be used When the major source of protein is potato protein having a molecular mass greater than 35 kD, the carbohydrate comprises lactose and optionally saccharose, maltodextrin or starch.

In one embodiment, the carbohydrate comprises maltodextrin. In one embodiment, at least 40%, 50%, 60% or 70% by weight of the total carbohydrate is maltodextrin.

In one embodiment, the carbohydrate comprises lactose. In one embodiment, at least 40%, 50%, 60% or 70% by weight of the total carbohydrate is lactose.

In one embodiment, the carbohydrate comprises lactose and maltodextrin.

### Fat

The fat content of the infant formula of the invention is preferably in the range 4.0-6.0 g lipids per 100 kcal.

The fat may be any lipid or fat which is suitable for use in infant formula.

Example fats for use in the infant formula of the invention include sunflower oil, low erucic acid rapeseed oil, safflower oil, canola oil, olive oil, coconut oil, palm kernel oil, soybean oil, fish oil, palm oleic, high oleic sunflower oil and high oleic safflower oil, and microbial fermentation oil containing long chain, polyunsaturated fatty acids.

The fat may also be in the form of fractions derived from these oils, such as palm olein, medium chain triglycerides and esters of fatty acids such as arachidonic acid, linoleic acid, palmitic acid, stearic acid, docosahexaeonic acid, linolenic acid, oleic acid, lauric acid, capric acid, caprylic acid, caproic acid, and the like.

Further example fats include structured lipids (i.e. lipids that are modified chemically or enzymatically in order to change their structure). Preferably, the structured lipids are sn2 structured lipids, for example comprising triglycerides having an elevated level of palmitic acid at the sn2 position of the triglyceride.

Oils containing high quantities of preformed arachidonic acid and/or docosahexaenoic acid, such as fish oils or microbial oils, may also be added.

Long chain polyunsaturated fatty acids, such as dihomo-γ-linolenic acid, arachidonic acid, eicosapentaenoic acid and docosahexaenoic acid, may be added. Willemsen et al. showed that the addition of such fatty acids supported epithelial barrier integrity and reduced IL-4 mediated permeability (Willemsen, L.E. et al. (2008) Eur. J. Nutr. 47: 183-91).

Structured lipids may be added or may be omitted. Medium chain triglycerides may be added or may be omitted.

### Further ingredients

The infant formula of the invention preferably also contains all vitamins and minerals understood to be essential in the daily diet in nutritionally significant amounts. Minimum requirements have been established for certain vitamins and minerals.

Example vitamins, minerals and other nutrients for use in the infant formula of the invention include vitamin A, vitamin B1, vitamin B2, vitamin B6, vitamin B12, vitamin E, vitamin K, vitamin C, vitamin D, folic acid, inositol, niacin, biotin, pantothenic acid, choline, calcium, phosphorous, iodine, iron, magnesium, copper, zinc, manganese, chlorine, potassium, sodium, selenium, chromium, molybdenum, taurine and L-carnitine.

Minerals are usually added in their salt form.

The infant formula of the invention may also comprise at least one probiotic. The term "probiotic" refers to microbial cell preparations or components of microbial cells with beneficial effects on the health or well-being of the host (Salminen, S. et al. (1999) Trends Food Sci. Technol. 10: 107-10).

In particular, probiotics may improve gut barrier function (Rao, R.K. (2013) Curr. Nutr. Food Sci. 9: 99-107).

Preferred probiotics are those which as a whole are safe, are L(+) lactic acid producing cultures and have acceptable shelf-life for products that are required to remain stable and effective for up to 24 months.

Examples of probiotic micro-organisms for use in the infant formula of the invention include yeasts, such as *Saccharomyces, Debaromyces, Candida, Pichia* and *Torulopsis;* and bacteria, such as the genera *Bifidobacterium, Bacteroides, Clostridium, Fusobacterium, Melissococcus, Propionibacterium, Streptococcus, Enterococcus, Lactococcus, Staphylococcus, Peptostrepococcus, Bacillus, Pediococcus, Micrococcus, Leuconostoc, Weissella, Aerococcus, Oenococcus* and *Lactobacillus.*

Specific examples of suitable probiotic microorganisms are: *Saccharomyces cereviseae, Bacillus coagulans, Bacillus licheniformis, Bacillus subtilis, Bifidobacterium bifidum, Bifidobacterium infantis, Bifidobacterium longum, Enterococcus faecium, Enterococcus faecalis, Lactobacillus acidophilus, Lactobacillus alimentarius, Lactobacillus casei* subsp. *casei, Lactobacillus casei Shirota, Lactobacillus curvatus, Lactobacillus delbruckii* subsp. *lactis, Lactobacillus farciminus, Lactobacillus gasseri, Lactobacillus helveticus, Lactobacillus johnsonii, Lactobacillus rhamnosus (Lactobacillus GG), Lactobacillus sake, Lactococcus lactis, Micrococcus varians, Pediococcus acidilactici, Pediococcus pentosaceus, Pediococcus acidilactici, Pediococcus halophilus, Streptococcus faecalis, Streptococcus thermophilus, Staphylococcus carnosus* and *Staphylococcus xylosus.*

Preferred probiotic bacterial strains include *Lactobacillus rhamnosus; Lactobacillus rhamnosus* LPR (CGMCC 1.3724); *Bifidobacterium lactis* BL818 (CNCM 1-3446) sold inter alia by the Christian Hansen company of Denmark under the trade mark BB 12; and *Bifidobacterium longum* BL999 (ATCC BAA-999) sold by Morinaga Milk Industry Co. Ltd. of Japan under the trade mark BB536.

The infant formula of the invention may also contain other substances which may have a beneficial effect such as human milk oligosaccharides, prebiotics, lactoferrin, fibres, nucleotides, nucleosides and the like.

### Method of manufacture

The infant formula of the invention may be prepared in any suitable manner. For example, it may be prepared by blending together the protein source, the carbohydrate source and the fat source in appropriate proportions. If used, the further emulsifiers may be included at this point. The vitamins and minerals may be added at this point but vitamins are usually added later to avoid thermal degradation. Any lipophilic vitamins, emulsifiers and the like may be dissolved in the fat source prior to blending. Water, preferably water which has been subjected to reverse osmosis, may then be mixed in to form a liquid mixture. Commercially available liquefiers may be used to form the liquid mixture. The liquid mixture may then be homogenised. The liquid mixture may then be thermally treated to reduce bacterial loads. This may be carried out, for example, by means of steam injection, or using an autoclave or heat exchanger, for example a plate heat exchanger. The liquid mixture may then be cooled and/or homogenised. The pH and solid content of the homogenised mixture may be adjusted at this point. The homogenised mixture may then be transferred to a suitable drying apparatus such as a spray dryer or freeze dryer and converted to powder. If a liquid infant formula is preferred, the homogenised mixture may be sterilised, then aseptically filled into a suitable container or may be first filled into a container and then retorted.

### EXAMPLES

### Example 1

### Nutritional comparison between potato protein and rice protein

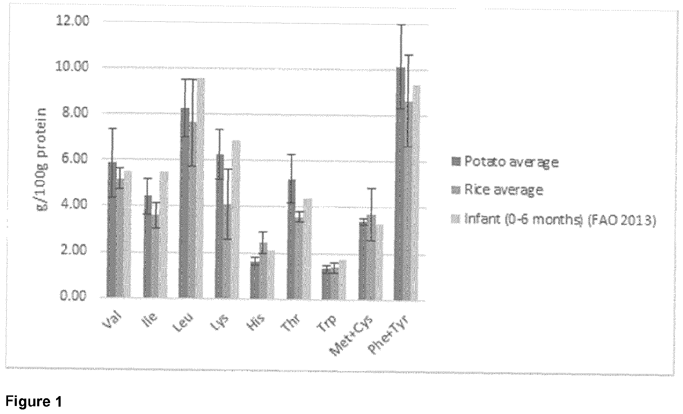

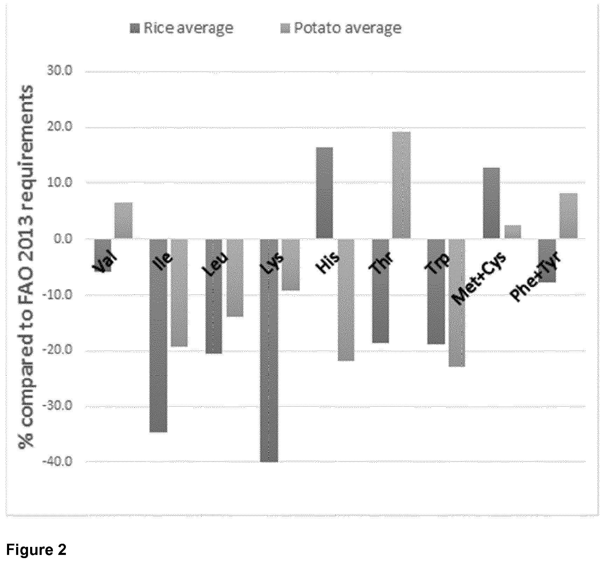

Potato protein contains higher levels of the following essential amino acids compared to rice protein (Figures 1 and 2): valine; isoleucine; leucine; lysine; threonine and aromatic amino acids.

The concentrations of tryptophan and the sulphur-containing amino acids are similar between potato and rice proteins.

However, rice protein contains higher concentrations of histidine than potato protein.

Overall, the essential amino acid concentrations in potato protein are better than rice protein, and may require lower levels of additional amino acid fortification.

Potato protein contains more essential amino acids in compliance with the FAO 2013 recommendations compared to rice protein (Table 1).

**Table 1. Amino acid concentrations in potato and rice proteins that are complaint with the FAO 2013 recommendations for 0-6 month-old infants**

| **Amino acid** | **Potato** | **Rice** |
|---|---|---|
| Val | ✔ | × |
| Ile | × | × |
| Leu | × | × |
| Lys | × | × |
| His | × | ✔ |
| Thr | ✔ | × |
| Trp | × | × |
| Met+Cys | ✔ | ✔ |
| Phe+Tyr | ✔ | × |

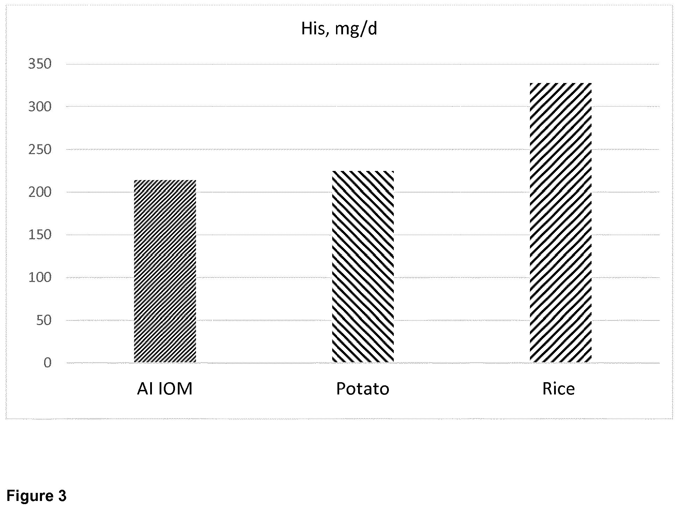

Although the levels of histidine are lower in potato protein than rice protein, and are lower than the FAO 2013 recommendations for 0-6 month-old infants, potato protein will still deliver histidine levels that are compliant with the 214 mg/d histidine suggested by Institute of Medicine of the National Academies Adequate Intake (Al for 0-6 month-old infants (Figure 3).

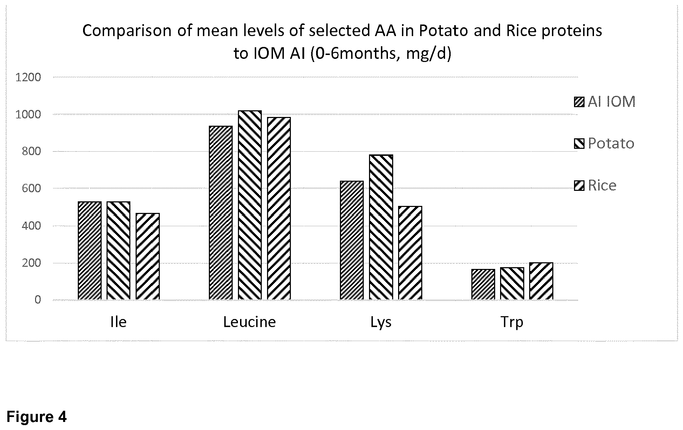

Furthermore, although the concentrations of isoleucine, leucine, lysine and tryptophan are lower in potato compared to the FAO 2013 recommendations, these levels are similar or higher than the levels in rice. Additionally, potato protein will meet the Institute of Medicine of the National Academies Al recommendations for these amino acids, while rice protein will not meet the recommendations for isoleucine and lysine (Figure 4).

The concentrations for isoleucine, leucine and lysine taken from the supplier data indicate the levels of these amino acids will be compliant with WHO 2007, 2013 and EC Directive 2006/141/EC, and codex standard (CODEX STAN 72-1981), in addition to Institute of Medicine of the National Academies Al recommendations.

### Branched-chain amino acids (BCAA)

Branched-chain amino acids (BCAA; leucine, isoleucine and valine) have an important role in protein synthesis. Leucine is an activator of mTOR, and promotes protein synthesis and suppress protein catabolism, resulting in maintenance of muscle protein during restricted dietary intake. Children with food allergies follow dietary restrictions, therefore they are at risk of developing malnutrition, hence consumption of plant protein with high levels of BCAAs may help maintain muscle proteins.

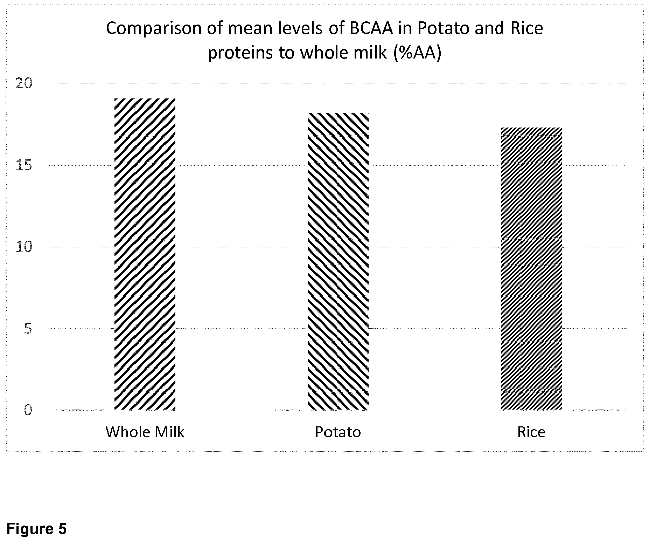

Additionally, the best food sources of BCAAs are meat, fish, dairy products and eggs, which may not be consumed at all, or at least consumed in smaller amounts by infants and small children with food allergies. Figure 5 shows that the sum of BCAA (%AA) in potato is closer to that in milk and therefore provides an advantage to children with cow's milk protein allergy. Accordingly, providing a protein source with higher levels of BCAAs may benefit this paediatric population.

### Lysine and threonine

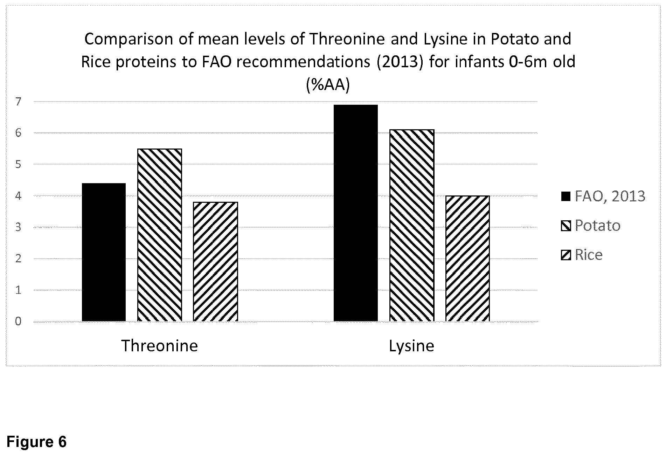

Lysine and threonine are the first and second most limiting amino acids, respectively, for protein synthesis in human subjects consuming a predominantly cereal-based diet such as wheat and rice. The main roles of lysine and threonine are in protein synthesis. Unlike other plant proteins sources such as rice and wheat proteins, potato protein has higher levels of these two amino acids, with lysine levels close to the requirement set by the FAO 2013 recommendations and threonine levels exceeding it (Figure 6).

The best food sources of threonine and lysine are soy, dairy products, nuts, and fish, beef or chicken. These food sources may not be consumed at all, or at least consumed in smaller amounts by infants and small children with food allergies. Therefore providing a non-animal source of protein with high concentrations of these two amino acids will benefit this paediatric population.

### Aromatic amino acids

Phenylalanine is a precursor for tyrosine, the neurotransmitters dopamine, norepinephrine, and adrenaline, and the skin pigment melanin. Potato protein exceeds the requirements set by the FAO 2013 recommendations for 0-6 month-old infants, while rice does not meet the recommended level.

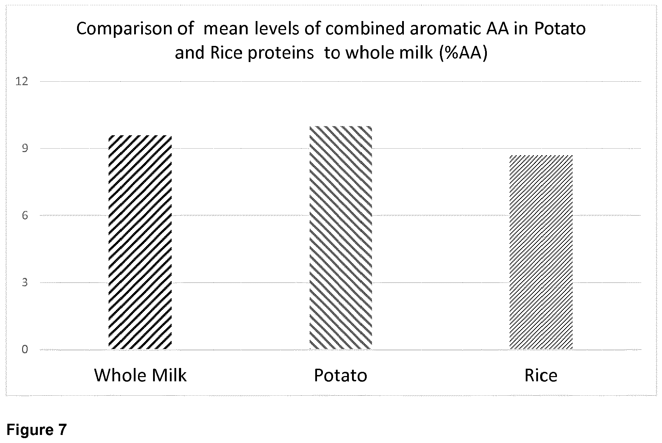

The best food sources of phenylalanine are eggs, chicken, liver, beef, milk and soybeans. These food sources may not be consumed at all, or at least consumed in smaller amounts by infants and small children with food allergies. However, the combined levels of phenylalanine and tyrosine in potato protein are similar to those in milk (Figure 7), which provides an advantage to infants and children with cow's milk protein allergy.

### Example 2

### Infant formula sensory evaluation

Infant formulas comprising potato protein were compared to a representative rice-based infant formula (Modilac) for sensory evaluation with a panel of 12 people (Table 2). Infant formula compositions with full range potato proteins are comparative.

**Table 2**

| | **Modilac rice 1** | **Infant formula with high molecular mass potato fraction (>35kDa)** (with and without lactose very close in sensory profile and therefore combined) | **Infant formula with full range potato proteins** (i.e. no molecular mass-based fractionation) **(with lactose)** | **infant formula with low molecular mass potato protein fraction (<35kDa) (with lactose)** | **Infant formula with hydrolysed potato protein** (full range potato proteins , i.e. no molecular mass-based fractionation) **(with lactose)** |
|---|---|---|---|---|---|
| **Appearance** | Brown | Yellow /White | Grey and foam | White like milk | Brown / grey Phase separation |
| **Odour** | Neutral. A bit bitter and chlorine / biny | Cereal / cooked potato | Cereal / moulty | neutral | Neutral A bit Potato / bouillon |
| **Flavour** | Bitter / hydrolysed/ plant and green taste / astringent | Cooked soy/ bitter / astringent / cereal / burned potato / vegetable | Cereal/ Flavour/ astringent/ vegetable / soya / sweet / soapy | Milky Cereal Astringent | Bitter / Astringent / Burned potato / cereal |
| **Texture** | Very viscous /thick /creamy /slimmy | Sandy / powdery /Thick / mouthdrying | Smooth | Milky / a bit sandy | Thin / mouth drying |
| **After sensations** | Bitter/ astringent | Astringent / sandy / mouth coating | Astringent / mouth coating | None | Slightly bitter |

The potato-based infant formulas were all ranked more favourably than the rice-based formula.

### Example 3

### Infant formula formulation

An infant formula may be formulated according to the recipe in Table 3.

**Table 3.**

| | |
|---|---|
| Caloric density | 0.6-1.0 kcal/mL |
| Protein | 1.8-3.2 g/100 kcal |
| Carbohydrate | 9-14 g/100 kcal |
| Fat | 4.0-6.0 g/100 kcal |
| Other solids (e.g. free amino acids) | 0-2.0% |
| Ash | 2-3% |
| Water | <3% |
| Optional probiotics | |

The infant formula may optionally contain lactose.

### Example 4

Turbiscan analysis was carried out on the samples shown in Table 4 (samples comprising "full-range" potato protein are comparative). to test the stability of the emulsions formed.

**Table 4. Samples analysed by Turbiscan.**

| **Short Name** | **Trial** | **Protein Source** |
|---|---|---|
| <35 kDa fraction evap | 23740.002 | Low molecular mass fraction (<35 kDa) including trypsin inhibitors, soluble at low pH, 90.8 g/100 g protein |
| <35 kDa fraction evap pH 6 | 23740.002 | Same as 23740.002, but pH was raised from 4.88 to 6.0 before analysing |
| | pH 6.0 | |
| >35 kDa fraction lacto evap | 23740.006 | High molecular mass fraction (>35 kDa), patatin, soluble at neutral pH, 84.4 g/100 g protein; lactose and maltodextrin |
| >35 kDa fraction no lacto evap | 23740.008 | High molecular mass fraction (>35 kDa), patatin, soluble at neutral pH, 84.4 g/100 g protein; only maltodextrin |
| Full range (sample 1) lacto evap | 23740.010 | Full range proteins (i.e. no molecular mass-based fractionation), soluble at neutral pH, 86.3 g/100 g protein |
| Full range (sample 2) hydro evap | 23740.012 | Full range proteins (i.e. no molecular mass-based fractionation), insoluble - slightly hydrolyzed (3 h) to improve solubility, 83.4 g/100 g protein |

### Materials and methods

14.0 g of the relevant sample powder was dissolved in 90 mL water at 40°C and stirred for 2 minutes on a magnetic stirrer. 20.0 mL was transferred to a measuring cuvette and the Turbiscan measurement was immediately started. Samples were measured on a regular basis during the day at room temperature and were stored at 4°C overnight.

### Results and discussion

Lowest emulsion stability (correlating with the highest values from the Turbiscan analysis) was observed for the second sample of the complete potato protein extract ("Full range (sample 2)"), as expected. This sample is stable for a few minutes, but starts sedimenting.

The experiments with the high molecular mass fraction (no lactose) and the first sample of the complete potato protein extract ("Full range (sample 1)") showed good performance. The high molecular mass fraction (with lactose) and the low molecular mass fraction at both pH show extremely good performance.

## Claims

1. An infant formula comprising protein, carbohydrate and fat, wherein the major source of protein is potato protein, wherein the potato protein has a molecular mass less than 35 kDa and wherein the carbohydrate comprises lactose, saccharose, maltodextrin or starch.

2. An infant formula comprising protein, carbohydrate and fat, wherein the major source of protein is potato protein, wherein the potato protein has a molecular mass greater than 35 kDa and wherein the carbohydrate comprises lactose and optionally saccharose, maltodextrin or starch.

3. The infant formula of claim 1 or 2, wherein at least about 75% by weight of the total protein is potato protein, preferably wherein 100% by weight of the total protein is potato protein.

4. The infant formula of any preceding claim, wherein the protein is intact protein, partially hydrolysed protein, or extensively hydrolysed protein.

5. The infant formula of any preceding claim, wherein the infant formula further comprises free amino acids.

6. The infant formula of any preceding claim, wherein the infant formula does not comprise a further emulsifier.

7. The infant formula of any preceding claim, wherein the infant formula is in the form of a powder, preferably in the form of a powder for reconstitution in a liquid.

8. The infant formula of any preceding claim, wherein the infant formula is in the form of a reconstituted infant formula.

9. The infant formula of claim 1 or any one of claims 3 to 8, wherein the infant formula comprises lactose.

10. The infant formula of any preceding claim, wherein the infant formula comprises:
a. 1.8-3.2 g protein per 100 kcal;
b. 9-14 g carbohydrate per 100 kcal; and
c. 4.0-6.0 g lipids per 100 kcal

11. Use of potato protein for the manufacture of an infant formula as claimed in any preceding claim, wherein the major source of protein in the infant formula is potato protein.

12. An infant formula according to any one of claims 1-10 for use in a method of treatment of cow's milk protein allergy in an infant wherein said infant is fed said infant formula.

## Patentansprüche

1. Säuglingsnahrung, umfassend Protein, Kohlenhydrat und Fett, wobei die Hauptquelle von Protein Kartoffelprotein ist, wobei das Kartoffelprotein eine Molekülmasse von weniger als 35 kDa aufweist und wobei das Kohlenhydrat Lactose, Saccharose, Maltodextrin oder Stärke umfasst.

2. Säuglingsnahrung, umfassend Protein, Kohlenhydrat und Fett, wobei die Hauptquelle von Protein Kartoffelprotein ist, wobei das Kartoffelprotein eine Molekülmasse von mehr als 35 kDa aufweist und wobei das Kohlenhydrat Lactose und optional Saccharose, Maltodextrin oder Stärke umfasst.

3. Säuglingsnahrung nach Anspruch 1 oder 2, wobei mindestens etwa 75 Gew.-% des Gesamtproteins Kartoffelprotein sind, vorzugsweise wobei 100 Gew.-% des Gesamtproteins Kartoffelprotein sind.

4. Säuglingsnahrung nach einem der vorstehenden Ansprüche, wobei das Protein intaktes Protein, teilweise hydrolysiertes Protein oder weitgehend hydrolysiertes Protein ist.

5. Säuglingsnahrung nach einem der vorstehenden Ansprüche, wobei die Säuglingsnahrung ferner freie Aminosäuren umfasst.

6. Säuglingsnahrung nach einem der vorstehenden Ansprüche, wobei die Säuglingsnahrung keinen weiteren Emulgator umfasst.

7. Säuglingsnahrung nach einem der vorstehenden Ansprüche, wobei die Säuglingsnahrung in der Form eines Pulvers, vorzugsweise in der Form eines Pulvers für eine Rekonstitution in einer Flüssigkeit, vorliegt.

8. Säuglingsnahrung nach einem der vorstehenden Ansprüche, wobei die Säuglingsnahrung in der Form einer rekonstituierten Säuglingsnahrung vorliegt.

9. Säuglingsnahrung nach Anspruch 1 oder einem der Ansprüche 3 bis 8, wobei die Säuglingsnahrung Lactose umfasst.

10. Säuglingsnahrung nach einem der vorstehenden Ansprüche, wobei die Säuglingsnahrung umfasst:
a. 1,8-3,2 g Protein pro 100 kcal;
b. 9-14 g Kohlenhydrat pro 100 kcal; und
c. 4,0-6,0 g Lipide pro 100 kcal

11. Verwendung von Kartoffelprotein für die Herstellung einer Säuglingsnahrung nach einem der vorstehenden Ansprüche, wobei die Hauptquelle von Protein in der Säuglingsnahrung Kartoffelprotein ist.

12. Säuglingsnahrung nach einem der Ansprüche 1 bis 10 zur Verwendung in einem Verfahren für eine Behandlung von Kuhmilchproteinallergie in einem Säugling, wobei dem Säugling die Säuglingsnahrung zugeführt wird.

## Revendications

1. Préparation pour nourrisson comprenant une protéine, un glucide et une matière grasse, dans laquelle la source majeure de protéine est une protéine de pomme de terre, dans laquelle la protéine de pomme de terre a une masse moléculaire inférieure à 35 kDa et dans laquelle le glucide comprend du lactose, du saccharose, de la maltodextrine ou de l'amidon.

2. Préparation pour nourrisson comprenant une protéine, un glucide et une matière grasse, dans laquelle la source majeure de protéine est une protéine de pomme de terre, dans laquelle la protéine de pomme de terre a une masse moléculaire supérieure à 35 kDa et dans laquelle le glucide comprend du lactose et facultativement du saccharose, de la maltodextrine ou de l'amidon.

3. Préparation pour nourrisson selon la revendication 1 ou 2, dans laquelle au moins environ 75 % en poids de la protéine totale est la protéine de pomme de terre, de préférence dans laquelle 100 % en poids de la protéine totale est la protéine de pomme de terre.

4. Préparation pour nourrisson selon l'une quelconque revendication précédente, dans laquelle la protéine est une protéine intacte, une protéine partiellement hydrolysée, ou une protéine hydrolysée de manière extensive.

5. Préparation pour nourrisson selon l'une quelconque revendication précédente, dans laquelle la préparation pour nourrisson comprend en outre des acides aminés libres.

6. Préparation pour nourrisson selon l'une quelconque revendication précédente, dans laquelle la préparation pour nourrisson ne comprend pas d'émulsifiant supplémentaire.

7. Préparation pour nourrisson selon l'une quelconque revendication précédente, dans laquelle la préparation pour nourrisson est sous la forme d'une poudre, de préférence sous la forme d'une poudre pour reconstitution dans un liquide.

8. Préparation pour nourrisson selon l'une quelconque revendication précédente, dans laquelle la préparation pour nourrisson est sous la forme d'une préparation pour nourrisson reconstituée.

9. Préparation pour nourrisson selon la revendication 1 ou selon l'une quelconque des revendications 3 à 8, dans laquelle la préparation pour nourrisson comprend du lactose.

10. Préparation pour nourrisson selon l'une quelconque revendication précédente, dans laquelle la préparation pour nourrisson comprend :
a. 1,8 à 3,2 g de protéine pour 100 kcal ;
b. 9 à 14 g de glucide pour 100 kcal ; et
c. 4,0 à 6,0 g de lipides pour 100 kcal

11. Utilisation de protéine de pomme de terre pour la fabrication d'une préparation pour nourrisson selon l'une quelconque revendication précédente, dans laquelle la source majeure de protéine dans la préparation pour nourrisson est une protéine de pomme de terre.

12. Préparation pour nourrisson selon l'une quelconque des revendications 1 à 10, destinée à être utilisée dans un procédé de traitement d'allergie aux protéines du lait de vache chez un nourrisson, dans laquelle ledit nourrisson est nourri avec ladite préparation pour nourrisson.
